# EUROPEAN PATENT APPLICATION

(11) **EP 3 735 895 A1**
(43) Date of publication of application: **11.11.2020**
(21) Application number: 19173826.9
(22) Date of filing: 10.05.2019
(51) Int. Cl.: A61B 5/0488, A61B 5/11, A61B 5/00

(54) **DETECTION OF PARALYSIS, WEAKNESS AND/OR NUMBNESS IN A PART OF A BODY OF A SUBJECT**

(71) Applicant: Koninklijke Philips N.V., 5656 AG Eindhoven (NL)
(72) Inventor: LUI, Wing Lam, 5656 AE Eindhoven (NL); GEURTS, Lucas Jacobus Franciscus, 5656 AE Eindhoven (NL); TALGORN, Elise Claude Valentine, 5656 AE Eindhoven (NL); VAN LEENGOED, Marleen Johanna Jacoba, 5656 AE Eindhoven (NL)
(74) Representative: de Haan, Poul Erik

(57) **Abstract**

There is provided a system (100) for detecting paralysis, weakness or numbness. A processor (102) acquires data obtained by a sensor (106) of a personal care device (104) used by a subject on a first part of their body and compares this data to reference data obtained by the sensor used by the subject on a second part of their body to determine whether the acquired data comprises an abnormality. The acquired data is indicative of a manner in which the subject used the device on the first part and/or a physiological property of that part. The reference data is indicative of a manner in which the subject used the device on the second part and/or a physiological property of that part. The processor detects paralysis, weakness or numbness in the first or a third part of the body of the subject when the acquired data comprises an abnormality.

## Description

### FIELD OF THE INVENTION

The disclosure relates to a system and a method for detecting paralysis, weakness and/or numbness in a part of a body of a subject.

### BACKGROUND OF THE INVENTION

Facial paralysis, weakness and/or numbness of a subject can be a sign of a potentially serious medical condition, such as a stroke, a transient ischemic attack (TIA), neurofibromatosis, brain damage, a brain or neck tumor, facial nerve damage, etc. Facial paralysis, weakness and/or numbness is a condition in which the facial muscles of a subject become paralyzed or numb. The facial muscles of the subject may appear to droop or become weak. There are different degrees of facial paralysis, weakness and/or numbness. For example, only the lower half of the face may be affected, one whole side of the face may be affected, or both sides of the face may be affected. Facial paralysis, weakness and/or numbness may cause abnormal facial expressions, e.g. due to damage to the nerves to the face.

Similar to facial paralysis, weakness and/or numbness, tongue paralysis, weakness and/or numbness can also be a warning sign of a potentially serious medical condition, such as a stroke, a TIA, a brain tumor, etc. Tongue paralysis, weakness and/or numbness can occur when the hypoglossal nerve is damaged. In the most common case, the tongue paralysis, weakness and/or numbness of the subject is on one side, which is known as unilateral or asymmetric paralysis, weakness and/or numbness. In the case of tongue paralysis on one side, when the subject sticks out their tongue, it will deviate or point toward the side of the paralysis (i.e. the part that is damaged). In less-common cases, complete tongue paralysis, weakness and/or numbness may occur. In the case of complete tongue paralysis, the subject loses all control of their tongue. In this case, the tongue of the subject may not appear deviated. During tongue paralysis, a subject may often experience numbness and/or tingling of the tongue. The subject may find it difficult to speak properly due to tongue paralysis, weakness and/or numbness.

However, while tongue paralysis, weakness and/or numbness can be a sign of a serious medical condition, tongue paralysis, weakness and/or numbness may not always heavily impede the activities of the subject and it is not uncommon for a subject to experience numbness and/or tingling of the tongue due to non-life-threatening medical conditions, such as anxiety, dental problems, etc. Similarly, while facial paralysis, weakness and/or numbness can be a sign of a serious medical condition, it is not uncommon for a subject to experience degrees of facial weakness and/or exhibit abnormal facial expressions due to non-life-threatening medical conditions, such as psychiatric conditions (e.g. psychotic disorders, where the subject's sense of reality is impaired), stress, fatigue, Tourette's syndrome, Bell's palsy, a middle ear infection, etc. Also, in some situations where facial paralysis, weakness and/or numbness is in fact a sign of a serious medical condition (e.g. a TIA), the feeling of numbness and tightness around the face of the subject may only be temporary and may disappear after a short while. Thus, even though facial paralysis, weakness and/or numbness and tongue paralysis, weakness and/or numbness can be a sign of a serious medical condition, the potential hazards associated with them can often be unnoticed, overlooked, ignored, or not taken seriously by the subject. This can be problematic since an early medical intervention may be needed to treat an underlying serious medical condition and the subject may fail to seek such a medical intervention.

A non-invasive method to aid a subject in detecting signs of facial paralysis in everyday life involves using a smart phone with image-processing techniques to analyze facial expression and therefore quantify facial nerve paralysis. However, this method requires that the subject deliberately takes a close up photo of themselves at specific angle to have the facial paralysis analyzed. There also exists a method for detecting a tongue deviation angle as a warning sign of a stroke. However, this requires the subject to stick out their tongue for images of the tongue to be acquired and the degree of tongue deviation analyzed. Thus, existing techniques that make use of imaging technology to analyze a paralysis conditions require a deliberate action on the part of the subject for the technique to be implemented. Moreover, in order to identify relevant trends and corresponding medical conditions before they become critical, images need to be acquired on a daily basis, which can be inconvenient for the subject. Furthermore, the images have to be acquired at a specific angle, which can be cumbersome for the subject, and under certain environmental (e.g. light) conditions, which may be difficult. It is thus unlikely that subjects will be willing to perform such cumbersome and/or difficult image acquisition tasks on a regular basis, especially when the goal is to detect a medical condition of which they are not aware.

Other existing techniques include that which is described in US 8,493,286, where an apparatus measures movements of facial muscles in the face of a subject and a lack or impairment of the movement of the facial muscles can reveal facial paralysis. However, this existing technique requires facial movement sensors to be attached to (or secured in contact with) the skin on the face of the subject at various locations or even surgically implanted in the face of the subject at various locations. Thus, the technique can be inconvenient, uncomfortable, and/or invasive for the subject.

### SUMMARY OF THE INVENTION

As noted above, the limitations with existing techniques is that the existing techniques either require a subject to perform cumbersome and/or difficult image acquisition tasks on a regular basis or they require sensors to be attached to (or secured in contact with) the skin of the subject at various locations or even surgically implanted in the subject at various locations, which can be inconvenient, uncomfortable, and/or invasive for the subject. It would thus be valuable to have an improvement aimed at addressing these limitations.

Therefore, according to a first aspect, there is provided a system for detecting paralysis, weakness and/or numbness in a part of a body of a subject. The system comprising a processor configured to acquire data obtained by a sensor of a personal care device used by a subject on a first part of a body of a subject. The acquired data is indicative of a manner in which the subject used the personal care device on the first part of the body of the subject and/or a physiological property of the first part of the body of the subject. The processor is configured to compare the acquired data to reference data obtained by the sensor of the personal care device used by the subject on a second part of the body of the subject to determine whether the acquired data comprises an abnormality. The reference data is indicative of a manner in which the subject used the personal care device on the second part of the body of the subject and/or a physiological property of the second part of the body of the subject. The processor is configured to detect paralysis, weakness and/or numbness in the first part of the body of the subject or a third part of the body of the subject when the acquired data is determined to comprise an abnormality.

In some embodiments, the first part of the body of the subject and the second part of the body of the subject may be the same part of the body of the subject. In some embodiments, the first part of the body of the subject and the second part of the body of the subject may be corresponding parts of the body of the subject on opposite sides of the body.

In some embodiments, the processor may be configured to detect partial paralysis, weakness and/or numbness in the first part of the body of the subject when the acquired data is determined to comprise an abnormality.

In some embodiments, the third part of the body of the subject may be a different part of the body of the subject to the first and second parts of the body of the subject.

In some embodiments, the reference data may comprise previous and/or subsequent data obtained by the sensor of the personal care device used by the subject on the second part of the body of the subject.

In some embodiments, the processor may be configured to determine that the acquired data comprises an abnormality if the acquired data differs from the reference data by more than a predefined amount.

In some embodiments, the processor may be configured to, if paralysis, weakness and/or numbness is detected in the first part of the body of the subject, output any one or more of: an indication of the detected paralysis, weakness and/or numbness, an indication that the subject is at an increased risk of a medical condition, and a request for information from the subject.

In some embodiments, the system may comprise the personal care device comprising the sensor. In some embodiments, the sensor may be configured to obtain the data while the subject uses the personal care device on the first part of the body of the subject and obtain the reference data while the subject uses the personal care device on the second part of the body of the subject. In some embodiments, the personal care device may comprise the processor.

In some embodiments, the manner in which the subject uses the personal care device may comprise any one or more of: the manner in which the subject holds the personal care device, the direction in which the subject uses the personal care device, the manner in which the subject moves the personal care device, the speed at which the subject moves the personal care device, the pressure or force with which the subject uses the personal care device, and the orientation at which the subject uses the personal care device.

In some embodiments, the physiological property may comprise any one or more of: a muscle tension, a muscle elasticity, a skin morphology, and a skin and/or tongue orientation.

In some embodiments, the paralysis, weakness and/or numbness may comprise any one or more of: a facial paralysis, weakness and/or numbness; a tongue paralysis, weakness and/or numbness; and a hand paralysis, weakness and/or numbness.

According to a second aspect, there is provided a method for detecting paralysis, weakness and/or numbness in a part of a body of a subject. The method comprises acquiring data obtained by a sensor of a personal care device used by a subject on a first part of a body of a subject. The acquired data is indicative of a manner in which the subject used the personal care device on the first part of the body of the subject and/or a physiological property of the first part of the body of the subject. The method comprises comparing the acquired data to reference data obtained by the sensor of the personal care device used by the subject on a second part of the body of the subject to determine whether the acquired data comprises an abnormality. The reference data is indicative of a manner in which the subject used the personal care device on a second part of the body of the subject and/or a physiological property of the second part of the body of the subject. The method comprises detecting paralysis, weakness and/or numbness in the first part of the body of the subject or a third part of the body of the subject when the acquired data is determined to comprise an abnormality.

According to a third aspect, there is provided a computer program product comprising a computer readable medium. The computer readable medium has computer readable code embodied therein. The computer readable code is configured such that, on execution by a suitable computer or processor, the computer or processor is caused to perform the method described earlier.

According to the aspects and embodiments described above, the limitations of existing techniques are addressed. In particular, according to the above-described aspects and embodiments, paralysis, weakness and/or numbness in a part of the body of a subject can be detected whilst relieving the burden on the subject. In particular, the subject is not required to perform cumbersome and/or difficult image acquisition tasks on a regular basis for the paralysis, weakness and/or numbness detection. Instead, paralysis, weakness and/or numbness can be detected as the subject goes about their daily routine without the subject being required to perform any extra actions. In this way, the paralysis, weakness and/or numbness detection can be performed without interfering with the activities of the subject. The detection of paralysis, weakness and/or numbness according to the above-described aspects and embodiments is convenient, comfortable, and non-invasive for the subject. There is thus provided an improved system and method for detecting paralysis, weakness and/or numbness in a part of the body of a subject.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a schematic illustration of a system according to an embodiment;
Fig. 2 is a flow chart illustrating a method according to an embodiment; and
Fig. 3 is a flow chart illustrating a method according to an embodiment.

### DETAILED DESCRIPTION OF EMBODIMENTS

As noted above, there is provided herein an improved system and method for detecting paralysis, weakness and/or numbness in a part of a body of a subject. Herein, the paralysis, weakness and/or numbness may comprise any one or more of a facial paralysis, weakness and/or numbness, a tongue paralysis, weakness and/or numbness, a hand paralysis, weakness and/or numbness, and any other paralysis, weakness and/or numbness.

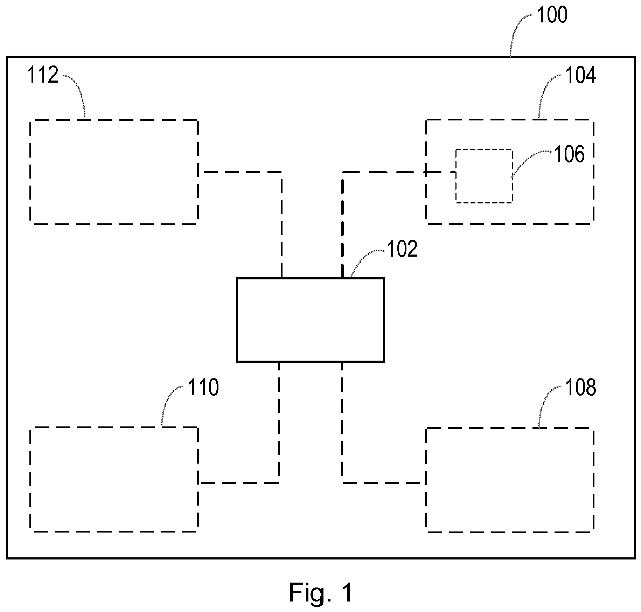

Fig. 1 illustrates a system 100 for detecting paralysis, weakness and/or numbness in a part of a body of a subject according to an embodiment. As illustrated in Fig. 1, the system 100 comprises a processor 102. The processor 102 can be implemented in numerous ways, with software and/or hardware, to perform the various functions described herein.

In particular implementations, the processor 102 can comprise a plurality of software and/or hardware modules, each configured to perform, or that are for performing, individual or multiple steps of the method described herein. The processor 102 may comprise one or more processors (e.g. one or more microprocessors, one or more multi-core processors and/or one or more digital signal processors (DSPs)), one or more processing units, and/or one or more controllers (such as one or more microcontrollers) that may be configured or programmed (e.g. using software or computer program code) to perform the various functions described herein. The processor 102 may be implemented as a combination of dedicated hardware (e.g. amplifiers, pre-amplifiers, analog-to-digital convertors (ADCs) and/or digital-to-analog convertors (DACs)) to perform some functions and a processor (e.g. one or more programmed microprocessors, DSPs and associated circuitry) to perform other functions.

Briefly, the processor 102 is configured to acquire data obtained by a sensor 106 of a personal care device 104 used by a subject on a first part of a body of a subject. The acquired data is indicative of a manner in which the subject used the personal care device on the first part of the body of the subject and/or a physiological property of the first part of the body of the subject. The processor 102 is also configured to compare the acquired data to reference data obtained by the sensor 106 of the personal care device 104 used by the subject on a second part of the body of the subject to determine whether the acquired data comprises (or to determine whether to classify the data as comprising) an abnormality. The reference data is indicative of a manner in which the subject used the personal care device on a second part of the body of the subject and/or a physiological property of the second part of the body of the subject. The processor 102 is configured to detect paralysis, weakness and/or numbness in the first part of the body of the subject or a third part of the body of the subject when the acquired data is determined to comprise (or is classified as comprising) an abnormality.

As illustrated in Fig. 1, in some embodiments, the system 100 can comprise the personal care device 104 comprising the sensor 106. As illustrated in Fig. 1, in some embodiments, the processor 102 may be external to (e.g. separate to or remote from) the personal care device 104 comprising the sensor 106. For example, another device may comprise the processor 102 according to some embodiments. However, in other embodiments (not illustrated), the personal care device 104 comprising the sensor 106 may also comprise the processor 102.

The personal care device 104 described herein can by any personal care device. For example, the personal care device 104 may be any device used in personal care, e.g. any device used in personal hygiene and/or beautification. The personal care device 104 can be an electronic or electric personal care device. The personal care device 104 can be for use on any part of a body of a subject, such as on the face of the subject, in the mouth of the subject, on the tongue of the subject, or any other part of the body of the subject. The personal care device 104 can be a handheld personal care device 104. That is, the personal care device 104 can be operable to be held by the subject in use (e.g. in the right hand of the subject or in the left hand of the subject). Examples of a personal care device 104 include, but are not limited to, a toothbrush, a tongue cleaner, an air floss device, a skin care device (e.g. a cleansing device, a skin treatment device, a facial clean brush, a face massage device, a rejuvenation device, or any other skin care device), a grooming device (e.g. a hairdryer, a brush, a hair removal device, a shaver, hair trimmers, hair clippers, or any other grooming device), or any other personal care device. The personal care device 104 may be used as part of a regular (e.g. daily) routine of the subject.

As mentioned earlier, the personal care device 104 comprises a sensor 106. In some embodiments, the sensor 106 may be embedded in the personal care device 104. The sensor 106 of the personal care device 104 is configured to obtain the data indicative of the manner in which the subject used the personal care device on the first part of the body of the subject and/or the physiological property of the first part of the body of the subject. Thus, the sensor 106 is configured to obtain the data while the subject uses the personal care device 104 on the first part of the body of the subject. Similarly, the sensor 106 is configured to obtain the reference data indicative of the manner in which the subject used the personal care device on the second part of the body of the subject and/or the physiological property of the second part of the body of the subject. Thus, the sensor 106 is configured to obtain the reference data while the subject uses the personal care device 104 on the second part of the body of the subject. The sensor 106 referred to herein may be a single sensor or a plurality (e.g. a series or array) of sensors.

Examples of the sensor 106 include, but are not limited to, a muscle tension sensor (e.g. an electromyography (EMG) sensor, or any other sensor, or any combination of sensors, suitable for measuring muscle tension), a muscle contraction sensor (e.g. an electro-optical muscle contraction sensor, a mechanomyogram (MMG) sensor, an ultrasound sensor such as a capacitive micromachined ultrasonic transducer (CMUT), or any other sensor, or any combination sensors, suitable for measuring muscle contraction), a motion sensor (e.g. a passive infrared (PIR) sensor, an ultrasonic sensor, an accelerometer, a gyroscope, or any other sensor, or any combination of sensors, suitable for measuring motion), an orientation sensor (e.g. a gyroscope, an accelerometer such as a three-axis accelerometer, or any other sensor, or any combination of sensors, suitable for measuring orientation), a force or pressure sensor (e.g. a strain gauge such as a piezoresistive strain gauge, a capacitive sensor, an optical sensor, or any other sensor, or any combination of sensors, suitable for measuring force and/or pressure), or any other sensor or combination of sensors, suitable for obtaining data indicative of the manner in which the subject used the personal care device 104 on a part of a body of a subject and/or the physiological property of the part of the body of the subject. A person skilled in the art will be aware of a variety of sensors 106 suitable for obtaining data indicative of the manner in which the subject used the personal care device 104 on a part of a body of a subject and/or the physiological property of the part of the body of the subject and the manner in which those sensors may be configured to operate to obtain the data.

As illustrated in Fig. 1, in some embodiments, the system 100 may comprise at least one memory 108. In some embodiments, as illustrated in Fig. 1, at least one memory 108 may be external to (e.g. separate to or remote from) the personal care device 104 comprising the sensor 106. For example, another device may comprise at least one memory 108 according to some embodiments. In some embodiments, a hospital database may comprise at least one memory 108, at least one memory 108 may be a cloud computing resource, or similar. Alternatively or in addition to at least one memory 108 being external to (e.g. separate to or remote from) the personal care device 104, in some embodiments (not illustrated), the personal care device 104 may comprise at least one memory 108.

The processor 102 of the system 100 may be configured to communicate with and/or connect to at least one memory 108. A memory 108 may comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM), and electrically erasable PROM (EEPROM). In some embodiments, at least one memory 108 can be configured to store program code that can be executed by the processor 102 of the system 100 to cause the system 100 to operate in the manner described herein.

Alternatively or in addition, in some embodiments, at least one memory 108 can be configured to store information required by or resulting from the method described herein. For example, in some embodiments, at least one memory 108 may be configured to store any one or more of the acquired data, the reference data, an indication of whether the acquired data is determined to comprise (or is classified as comprising) an abnormality, an indication of whether paralysis, weakness and/or numbness is detected in the first part of the body of the subject or a third part of the body of the subject, or any other information, or any combination of information, required by or resulting from the method described herein. In some embodiments, the processor 102 of the system 100 can be configured to control at least one memory 108 to store information required by or resulting from the method described herein.

As illustrated in Fig. 1, in some embodiments, the system 100 may comprise at least one user interface 110. In some embodiments, as illustrated in Fig. 1, at least one user interface 110 may be external to (e.g. separate to or remote from) the personal care device 104 comprising the sensor 106. For example, another device may comprise at least one user interface 110 according to some embodiments. Alternatively or in addition to at least one user interface 110 being external to (e.g. separate to or remote from) the personal care device 104, in some embodiments (not illustrated), the personal care device 104 may comprise at least one user interface 110. The processor 102 of the system 100 may be configured to communicate with and/or connect to at least one user interface 110. In some embodiments, the processor 102 of the system 100 can be configured to control at least one user interface 110 to operate in the manner described herein.

A user interface 110 can be configured to render (or output, display, or provide) information required by or resulting from the method described herein. For example, in some embodiments, the user interface 110 may be configured to render (or output, display, or provide) any one or more of the acquired data, the reference data, an indication of whether the acquired data is determined to comprise (or is classified as comprising) an abnormality, an indication of whether paralysis, weakness and/or numbness is detected in the first part of the body of the subject or a third part of the body of the subject, or any other information, or any combination of information, required by or resulting from the method described herein. Alternatively or in addition, the user interface 110 can be configured to receive a user input. For example, the user interface 110 may allow a user (e.g. the subject or another user) to manually enter information or instructions, interact with and/or control the system 100. Thus, the user interface 110 may be any user interface that enables the rendering (or outputting, displaying, or providing) of information and/or enables a user to provide a user input.

For example, the user interface 110 may comprise one or more switches, one or more buttons, a keypad, a keyboard, a mouse, a display or display screen, a graphical user interface (GUI) such as a touch screen, an application (e.g. on the personal care device, a smart device such as a tablet, a smartphone, or any other smart device), or any other visual component, one or more speakers, one or more microphones or any other audio component, one or more lights (e.g. one or more light emitting diodes, LEDs), a component for providing tactile or haptic feedback (e.g. a vibration function, or any other tactile feedback component), an augmented reality device (e.g. augmented reality glasses, or any other augmented reality device), a smart device (e.g. a smart mirror, a tablet, a smart phone, a smart watch, or any other smart device), or any other user interface, or combination of user interfaces. In some embodiments, the user interface that is controlled to render information may be the same user interface as that which enables the user to provide a user input.

As illustrated in Fig. 1, in some embodiments, the system 100 may comprise at least one communications interface (or communications circuitry) 112. In some embodiments, as illustrated in Fig. 1, at least one communications interface 112 may be external to (e.g. separate to or remote from) the personal care device 104 comprising the sensor 106. For example, another device may comprise at least one communications interface 112 according to some embodiments. Alternatively or in addition to at least one communications interface 112 being external to (e.g. separate to or remote from) the personal care device 104, in some embodiments (not illustrated), the personal care device 104 may comprise at least one communications interface 112. A communications interface 112 can be for enabling the system 100, or components of the system 100 (e.g. the processor 102, the personal care device 104, the sensor 106 of the personal care device 104, at least one memory 108, at least one user interface 110 and/or any other components of the system 100), to communicate with and/or connect to each other and/or one or more other components. For example, a communications interface 112 can be for enabling the processor 102 of the system 100 to communicate with and/or connect to any one or more of the personal care device 104, the sensor 106 of the personal care device 104, at least one memory 108, at least one user interface 110 and/or any other components of the system 100.

A communications interface 112 may enable the system 100, or components of the system 100, to communicate and/or connect in any suitable way. For example, a communications interface 112 may enable the system 100, or components of the system 100, to communicate and/or connect wirelessly, via a wired connection, or via any other communication (or data transfer) mechanism. In some wireless embodiments, for example, the communications interface 112 may enable the system 100, or components of the system 100, to use radio frequency (RF), Bluetooth, or any other wireless communication technology to communicate and/or connect.

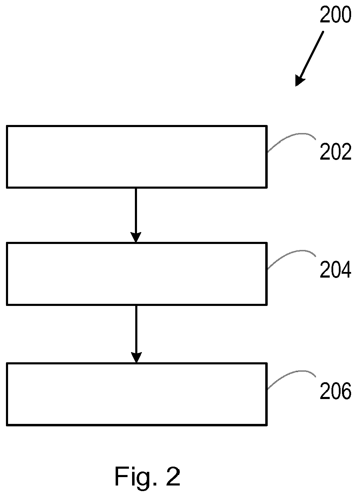

Fig. 2 illustrates a method 200 for detecting paralysis, weakness and/or numbness in a part of a body of a subject according to an embodiment. More specifically, Fig. 2 illustrates a method 200 of operating the system 100 described herein for detecting paralysis, weakness and/or numbness in a part of a body of a subject. The method 200 illustrated in Fig. 2 is a computer-implemented method. As described earlier, the system 100 comprises a processor 102. The method 200 illustrated in Fig. 2 can generally be performed by or under the control of the processor 102 of the system 100.

With reference to Fig. 2, at block 202 of Fig. 2, data obtained by a sensor 106 of a personal care device 104 used by a subject on a first part of a body of a subject is acquired. More specifically, the processor 102 of the system 100 acquires the data. As described earlier, the data is indicative of a manner in which the subject used the personal care device 104 on the first part of the body of the subject and/or a physiological property of the first part of the body of the subject. Herein, the manner in which the subject uses the personal care device 104 may comprise any one or more of the manner in which the subject holds the personal care device 104, the direction in which the subject uses the personal care device 104, the manner in which the subject moves the personal care device 104, the speed at which the subject moves the personal care device 104, the pressure or force with which the subject uses the personal care device 104, and the orientation at which the subject uses the personal care device 104. Also, herein, references to the physiological property may comprise any one or more of a muscle tension, a muscle elasticity, a skin morphology, a skin orientation (or angle), and a tongue orientation (or angle).

The sensor 106 of the personal care device 104 can be configured to obtain (or collect) the data as the personal care device 104 is used by the subject on the first part of the body of the subject. In some embodiments, the processor 102 of the system 100 may be configured to acquire the data from the sensor 106 of the personal care device 104 used by the subject on the first part of the body of the subject. Alternatively or in addition, in some embodiments, the data obtained by the sensor 106 of the personal care device 104 used by the subject on the first part of the body of the subject may be stored in one or more memories 108 of the system 100. In some of these embodiments, the processor 102 of the system 100 may be configured to acquire the data from the one or more memories 108 of the system 100.

Although not illustrated in Fig. 2, in some embodiments, the processor 102 of the system 100 may be configured to identify the first part of the body of the subject on which the personal care device 104 is used by the subject. That is, the processor 102 of the system 100 may be configured to identify on which part of the body of the subject the personal care device 104 is used by the subject. For example, the processor 102 of the system 100 may identify the first part of the body of the subject to be a left side of a face, a right side of the face, the entire face, a section of skin, or any other part of the body of the subject. In some embodiments, the processor 102 of the system 100 may be configured to identify the first part of the body of the subject using physiological feature recognition.

At block 204 of Fig. 2, the acquired data is compared to reference data obtained by the sensor 106 of the personal care device 104 used by the subject on a second part of the body of the subject to determine whether the acquired data comprises (or determine whether to classify the data as comprising) an abnormality. More specifically, the processor 102 of the system 100 compares the acquired data to reference data to determine whether the acquired data comprises an abnormality. As described earlier, the reference data is indicative of a manner in which the subject used the personal care device 104 on a second part of the body of the subject and/or a physiological property of the second part of the body of the subject. It may be assumed that the settings of the personal care device 104 when used on the second part of the body of the subject are the same as the settings of the personal care device 104 when used on the first part of the body of the subject.

As also mentioned earlier, herein, the manner in which the subject uses the personal care device 104 may comprise any one or more of the manner in which the subject holds the personal care device 104, the direction in which the subject uses the personal care device 104, the manner in which the subject moves the personal care device 104, the speed at which the subject moves the personal care device 104, the pressure or force with which the subject uses the personal care device 104, and the orientation at which the subject uses the personal care device 104. Also, herein, references to the physiological property may comprise any one or more of a muscle tension, a muscle elasticity, a skin morphology, a skin orientation (or angle), and a tongue orientation (or angle).

In some embodiments, the first part of the body of the subject and the second part of the body of the subject may be the same part of the body of the subject. For example, the first part of the body of the subject and the second part of the body of the subject may be the face of the subject, the skin of the subject, the mouth of the subject, the tongue of the subject, etc. In other embodiments, the first part of the body of the subject and the second part of the body of the subject are corresponding parts of the body of the subject on opposite sides of the body. For example, the first part of the body of the subject may be the right side of the face of the subject and the second part of the body of the subject may be the left side of the face of the subject, or vice versa. In another example, the first part of the body of the subject may be a right side of the mouth of the subject and the second part of the body of the subject may be a left side of the mouth of the subject, or vice versa. In yet another example, the first part of the body of the subject may be a right side of the tongue of the subject and the second part of the body of the subject may be a left side of the tongue of the subject, or vice versa.

The sensor 106 of the personal care device 104 can be configured to obtain (or collect) the reference data as the personal care device 104 is used by the subject on the second part of the body of the subject. In some embodiments, the processor 102 of the system 100 may be configured to acquire the reference data from the sensor 106 of the personal care device 104 used by the subject on the second part of the body of the subject. Alternatively or in addition, in some embodiments, the reference data obtained by the sensor 106 of the personal care device 104 used by the subject on the second part of the body of the subject may be stored in one or more memories 108 of the system 100. In some of these embodiments, the processor 102 of the system 100 may be configured to acquire the reference data from the one or more memories 108 of the system 100.

The reference data may comprise previous data obtained by the sensor 106 of the personal care device 104 used by the subject on the second part of the body of the subject. That is, the reference data may comprise data obtained by the sensor 106 of the personal care device 104 used by the subject on the second part of the body of the subject before (i.e. at an earlier time to) the personal care device 104 being used by the subject on the first part of the body of the subject. Alternatively or in addition, the reference data may comprise subsequent data obtained by the sensor 106 of the personal care device 104 used by the subject on the second part of the body of the subject. That is, the reference data may comprise data obtained by the sensor 106 of the personal care device 104 used by the subject on the second part of the body of the subject after (i.e. at a later time to) the personal care device 104 being used by the subject on the first part of the body of the subject. In some embodiments, the acquired data and the reference data may be data that is obtained by the sensor 106 of the personal care device 104 in the same (e.g. current) session of using the personal care device 104. In other embodiments, the acquired data and the reference data may be data that is obtained by the sensor 106 of the personal care device 104 in a different session of using the personal care device 104, e.g. the reference data may be data that is obtained by the sensor 106 of the personal care device 104 in a previous session of using the personal care device 104.

In some embodiments, the acquired data may comprise a map of the physiological property of the first part of the body of the subject and the reference data may comprise a map of the physiological property of the second part of the body of the subject. In these embodiments, the map of the physiological property of the first part of the body of the subject can be compared to the map of the physiological property of the second part of the body of the subject to determine whether the acquired data comprises (or determine whether to classify the data as comprising) an abnormality. In these embodiments, it may be determined that the acquired data comprises (or the acquired data may be classified as comprising) an abnormality if there are differences between the maps.

In some embodiments, where the first part of the body of the subject and the second part of the body of the subject are corresponding parts of the body of the subject on opposite sides of the body, the sensor 106 of the personal care device 104 may be configured to detect whether the personal care device 104 switches from being used by the subject on the first part of the body (e.g. the right side of the face) of the subject to being used by the subject on the second part of the body (e.g. the left side of the face) of the subject. In these embodiments, the sensor 106 of the personal care device 104 can be configured to continue to obtain the data to be compared to the reference data until the sensor 106 of the personal care device 104 detects that the personal care device 104 switches from being used by the subject on the first part of the body (e.g. the right side of the face) of the subject to being used by the subject on the second part of the body (e.g. the left side of the face) of the subject. Also, in these embodiments, the sensor 106 of the personal care device 104 can be configured to obtain the reference data when the sensor 106 of the personal care device 104 detects that the personal care device 104 switches from being used by the subject on the first part of the body (e.g. the right side of the face) of the subject to being used by the subject on the second part of the body (e.g. the left side of the face) of the subject.

In some embodiments, the sensor 106 of the personal care device 104 may be configured to continue to obtain the reference data until the personal care device 104 is no longer in use by the subject, e.g. until the routine of the subject using the personal care device 104 ends or finishes. In some embodiments where the sensor 106 of the personal care device 104 is configured to continue to acquire the reference data until the personal care device 104 is no longer in use by the subject, the processor 102 of the system 100 may be configured to compare the acquired data to the reference data when the personal care device 104 is no longer in use by the subject to determine whether the acquired data comprises (or determine whether to classify the data as comprising) an abnormality.

In some embodiments, the processor 102 can be configured to determine that the acquired data comprises (or classify the data as comprising) an abnormality if the comparison of the acquired data to the reference data indicates that there are asymmetric changes and/or changes over time. For example, the comparison of the acquired data to the reference data may indicate asymmetric changes and/or changes over time in the manner in which the subject used the personal care device and/or the physiological property. Thus, the comparison of the acquired data to the reference data may indicate asymmetric changes and/or changes over time in any one or more of: the manner in which the subject holds the personal care device 104, the direction in which the subject uses the personal care device 104, the manner in which the subject moves the personal care device 104, the speed at which the subject moves the personal care device 104, the pressure or force with which the subject uses the personal care device 104, the orientation at which the subject uses the personal care device 104, the muscle tension, the muscle elasticity, the skin morphology, a skin orientation (or angle), and the tongue orientation (or angle).

Herein, asymmetric changes may be those changes in the acquired data obtained from the first part of the body of the subject compared to the reference data obtained from the second part of the body of the subject, where the first and second parts of the body are corresponding parts of the body of the subject on opposite sides of the body (e.g. opposite sides of the face). Also, herein, changes over time may be those changes in the acquired data obtained from the first part of the body of the subject compared to the reference data obtained from the second part of the body of the subject at different times. In some embodiments, the processor 102 can be configured to determine that the acquired data comprises (or classify the data as comprising) an abnormality if the acquired data differs from the reference data by more than a predefined amount.

In some embodiments, the processor 102 can be configured to determine that the acquired data comprises (or classify the data as comprising) an abnormality if a pattern identified in the acquired data differs from a pattern identified in the reference data or if a pattern identified in the reference data is absent in the acquired data. The reference data to which the acquired data is compared may comprise typical reference patterns according to some embodiments. For example, the manner in which a subject uses the personal care device 104 may form a regular pattern in the data and the processor 102 can be configured to determine that the acquired data comprises (or classify the data as comprising) an abnormality if an irregular pattern is identified or if a pattern is absent in the acquired data. Thus, in some embodiments, the acquired data may be determined to comprise (or may be classified as comprising) an abnormality if the subject performs routine movements in a different way. In some embodiments, the patterns may be motion patterns and thus the acquired data and the reference data may be obtained by one or more motion sensors of the personal care device 104. An example of such an embodiment is where the personal care device 104 comprises a face cleaning device, which is used in circular motions on the face. These circular motions on the face are classified as regular patterns.

In an example, the processor 102 can be configured to determine that the acquired data comprises (or classify the data as comprising) an abnormality if the comparison of the acquired data to the reference data indicates that there is a change in the manner in which the personal care device 104 is held by the subject on one side of the face (or tongue) of the subject compared to the other side of the face (or tongue) of the subject. This can indicate paralysis, weakness or numbness on only one side of the face (or tongue) of the subject, where the personal care device 104 is held in a different way over the weak side of the face. By comparing asymmetric differences, the processor 102 can deduce a partial paralysis, weakness or numbness of the face. Also, the usage data can be compared with previous use of the personal care device 104 to ensure that there is a significant difference, e.g. a difference of more than a predefined amount.

In another example, the processor 102 can be configured to determine that the acquired data comprises (or classify the data as comprising) an abnormality if the comparison of the acquired data to the reference data indicates that there is a change in an orientation of the tongue with respect to the personal care device 104 and/or a change in a force on the personal care device 104 from the tongue. The force on the personal care device 104 from the tongue may, for example, be detected based on the load (e.g. pressure) that is sensed by the sensor 106 of the personal care device 104. For example, the personal care device 104 may be a toothbrush and the head of the toothbrush may comprise a sensor 106. Typically, the tongue presses against a toothbrush in use and this can be analyzed through the comparison.

In another example, the processor 102 can be configured to determine that the acquired data comprises (or classify the data as comprising) an abnormality if the comparison of the acquired data to the reference data indicates that there is a change in a pressure (or force) with which the device is used, e.g. that there is an increase in the pressure (or force) with which the device is used, or that there is an absence of pressure (or force). The subject may apply the device with more pressure due to a loss in sensitivity of the part of the body (e.g. the face or tongue) on which they use the device. The personal care device 104 may comprise a force sensor configured to detect a change or an absence of pressure in these embodiments. The change in pressure may, for example, be on the skin or teeth of the subject. In another example, the processor 102 can be configured to determine that the acquired data (or classify the data as comprising) comprises an abnormality if the comparison of the acquired data to the reference data indicates that there is a change in an orientation (e.g. angle) at which the personal care device 104 is used or a change in the movement of the personal care device 104. This can occur when there is an unusual morphology of the part of the body on which the personal care device 104 is used, e.g. a drooping cheek, a tongue in a different position, etc.

In another example, the processor 102 can be configured to determine that the acquired data comprises (or classify the data as comprising) an abnormality if the comparison of the acquired data to the reference data indicates that the subject uses (or handles) the personal care device 104 in a different manner. For instance, a subject usually holds a personal care device 104 (e.g. a toothbrush) with their dominant hand and a subject will normally use their other hand if they experience paralysis, weakness and/or numbness in their dominant hand. If a right-handed subject (who would usually use their personal care device 104 in their right hand) experiences paralysis, weakness or numbness in their right hand, it is likely that the subject will then use their personal care device in their left hand. Similarly, if a left-handed subject (who would usually use their personal care device 104 in their left hand) experiences paralysis, weakness or numbness in their left hand, it is likely that the subject will then use their personal care device in their right hand. Alternatively, if a subject continues to use their personal care device 104 in the same hand as usual despite experiencing paralysis, weakness or numbness in that hand, it is unlikely that the subject will use that hand without experiencing problems. Thus, for example, the personal care device 104 may be held in a different way to previous times (e.g. at a different orientation or angle, with a different pressure or grip, etc.), may be moved in an illogical manner, and/or may be used with an awkward posture.

Thus, in some embodiments, the processor 102 can be configured to determine that the acquired data comprises (or classify the data as comprising) an abnormality if the comparison of the acquired data to the reference data indicates that the subject used the personal care device 104 with a different hand. In some embodiments, this may be indicated by a change in orientation (or angle) of the personal care device 104, which can be determined from a comparison of data obtained by an orientation sensor of the personal care device 104. In some embodiments, the acquired data and the reference data may be obtained by an accelerometer and a motion (or movement) pattern of the manner in which the subject holds the personal care device 104 may be compared to determine whether there is a change in the manner in which the subject holds the personal care device 104 (or whether the subject is holding the personal care device 104 differently, such as with a different hand).

In another example, a subject usually retains the same pattern when using a personal care device 104. The use of a personal care device 104 is often part of a daily routine and is performed intuitively. If a subject suffers from paralysis, weakness or numbness in a part of their body on which the personal care device 104 is used, the subject may have to hold their personal care device 104 differently. For example, a subject usually retains the same pattern when using a toothbrush to brush their teeth or a tongue cleaning device to clean their tongue, e.g. starting from left to right, or right to left, etc. If a subject suffers from paralysis, weakness or numbness in the face or tongue, the subject may have to hold their toothbrush or tongue cleaning device differently (such as at a different angle or in a different direction), e.g. due to the tongue being in the way or the subject being unable to open their mouth as they usually do. Thus, in some embodiments, the processor 102 can be configured to determine that the acquired data comprises (or classify the data as comprising) an abnormality if the comparison of the acquired data to the reference data indicates that the subject is holding their personal care device 104 differently.

In another example, the processor 102 can be configured to determine that the acquired data comprises (or classify the data as comprising) an abnormality if the comparison of the acquired data to the reference data indicates that the subject is holding the personal care device 104 in a different position. For example, this may be due to arm weakness, such as the subject not being able to lift their arm above a certain height or not being able to keep their arm in a certain position. This difference in position may be indicated by comparing motion sensor data (e.g. angle and variations) to previous motion patterns. Alternatively or in addition, this difference in position may be indicated by comparing orientation data and, for example, determining that the angle at which the personal care device 104 is applied to the first part of the body of the subject is lower than the angle with which the personal care device 104 is applied to the second part of the body of the subject. For example, this can indicate a weakness in the arm of the subject with which the personal care device 104 is used.

In another example, the processor 102 can be configured to determine that the acquired data comprises (or classify the data as comprising) an abnormality if the comparison of the acquired data to the reference data indicates that the subject used the personal care device 104 on the first part of their body with a different speed to that used on the second part of their body. For example, when using an electric toothbrush, movements are typically slow and follow each quadrant. These movements are performed with a certain speed and in a certain order. Thus, in some embodiments, changes in the speed and/or order of movements can be indicate an abnormality.

In another example, facial muscles have different levels of elasticity when a subject experiences facial paralysis, weakness or numbness. In an embodiment where the acquired data and the reference data is obtained by an electro-optical muscle contraction sensor where (e.g. infrared) light is directed towards the facial muscle and backscattering is observed, the muscle contraction can be detected by measuring the change that occurs between the light scattered in the direction parallel and perpendicular to the muscle cells due to the muscle contraction. Thus, in some embodiments, the processor 102 can be configured to determine that the acquired data comprises (or classify the data as comprising) an abnormality if the comparison of the acquired data to the reference data indicates that there is a change in the muscle contraction.

In another example, the processor 102 can be configured to determine that the acquired data comprises (or classify the data as comprising) an abnormality if the comparison of the acquired data to the reference data indicates that there is a change in muscle tension. For example, the acquired data and reference data that is compared may be obtained by a muscle tension sensor, such as an EMG sensor. In some embodiments, acquired data and reference data obtained by a sensor 106 of a personal care device 104 at specific points may be compared. In some embodiments, a change in muscle tension may be correlated with a change in motion of the personal care device 104 in order to determine that that the acquired data comprises (or classify the data as comprising) an abnormality.

Although not illustrated in Fig. 2, in some embodiments, in addition to comparing the acquired data to the reference data, the processor 102 of the system 100 may be configured to compare any differences found between the acquired data and the reference data to differences found between acquired data and reference data obtained during a previous routine (or session) in which the subject used the personal care device 104 comprising the sensor 106. In this way, false alarms can be reduced.

Alternatively or in addition, in some embodiments, the processor 102 of the system 100 may be configured to compare acquired data obtained from a sensor 106 of a personal care device 104 used on a first part of the body (e.g. one side of the face) of the subject and also acquired data obtained from a sensor 106 of a personal care device 104 used on a corresponding first part (e.g. the other side of the face) of the body of the subject to the reference data. In these embodiments, the processor 102 of the system 100 may be configured to determine that the acquired data comprises (or classify the acquired data as comprising) an abnormality provided that only one (not both) of the comparisons indicates a difference or a difference of more than a predefined amount.

That is, the processor 102 of the system 100 may be configured to determine that the acquired data comprises (or classify the acquired data as comprising) an abnormality if the acquired data obtained from a sensor 106 of a personal care device 104 used on the first part of the body of the subject differs from the reference data (e.g. by more than a predefined amount) or the acquired data obtained from a sensor 106 of a personal care device 104 used on the corresponding first part of the body of the subject differs from the reference data (e.g. by more than a predefined amount). This can reduce false positives since differences in respect of both the acquired data obtained from a sensor 106 of a personal care device 104 used on the first part of the body and the acquired data obtained from a sensor 106 of a personal care device 104 used on the corresponding first part of the body of the subject may be attributable to a factor other than paralysis, weakness and/or numbness, which typically only occur on one side of the body at a time, particularly in the case of a serious medical condition (such as a stroke).

At block 206 of Fig. 2, paralysis, weakness and/or numbness is detected in the first part of the body of the subject or a third part of the body of the subject when the acquired data is determined to comprise (or is classified as comprising) an abnormality. More specifically, the processor 102 of the system 100 detects paralysis, weakness and/or numbness in the first part of the body of the subject or a third part of the body of the subject when the acquired data is determined to comprise (or is classified as comprising) an abnormality. That is, paralysis, weakness and/or numbness is detected as being present in the first part of the body of the subject or a third part of the body of the subject when the acquired data is determined to comprise (or is classified as comprising) an abnormality. In some embodiments, the processor 102 can be configured to detect complete paralysis, weakness and/or numbness in the first part of the body of the subject when the acquired data is determined to comprise (or is classified as comprising) an abnormality. In other embodiments, the processor 102 can be configured to detect partial paralysis, weakness and/or numbness in the first part of the body of the subject when the acquired data is determined to comprise (or is classified as comprising) an abnormality.

As mentioned, paralysis, weakness and/or numbness is detected in the first part of the body of the subject or a third part of the body of the subject when the acquired data is determined to comprise (or is classified as comprising) an abnormality. The third part of the body of the subject is a different part of the body of the subject to the first and second parts of the body of the subject. That is, the third part of the body of the subject is a different part of the body of the subject to that on which the personal care device 104 is used. In an example embodiment, the first and second parts of the body of the subject on which the personal care device 104 is used may be the face of the subject and the third part of the body of the subject in which paralysis, weakness and/or numbness is detected may be a hand of the subject. For example, the processor 102 of the system 100 may determine from the comparison of the acquired data to the reference data that the subject is using the personal care device 104 with a different hand to normal and the processor 102 may thus detect paralysis, weakness and/or numbness in the hand that the subject normally uses. In some embodiments, the processor 102 of the system 100 may log (e.g. store in one or memories 108 of the system 100) the comparison of the acquired data to the reference data over time. In this way, the progression or evolution of the detected paralysis, weakness and/or numbness (and thus any underlying medical condition) can be monitored over time.

In some embodiments, the processor 102 can be configured to, if paralysis, weakness and/or numbness is detected in the first part of the body of the subject, provide an output. For example, the processor 102 can be configured to control at least one user interface 110 (as described earlier) to render the output. In some embodiments, the output may be provided via a smart app. The output may comprise, for example, any one or more of an indication of the detected paralysis, weakness and/or numbness, a request for information from the subject (e.g. questions and/or tests to determine whether the subject is experiencing symptoms such as dizziness, difficulty speaking, saliva drooling, etc.) in order to gather more information, an indication that the subject is at an increased risk of a medical condition (e.g. if there is an acute change detected), an indication instructing the subject to seek medical advice, and an alert to a medical professional (such as a care giver or doctor, e.g. if there is sign of an acute medical condition such as a stroke) of the detected paralysis, weakness and/or numbness. In some embodiments, the detection of paralysis, weakness and/or numbness may trigger a protocol for self-diagnosis, e.g. to check with questions and/or tests if there is a health issue. In this way, the subject and/or a medical professional is more aware of abnormal symptoms that the subject would otherwise have ignored or neglected to notice.

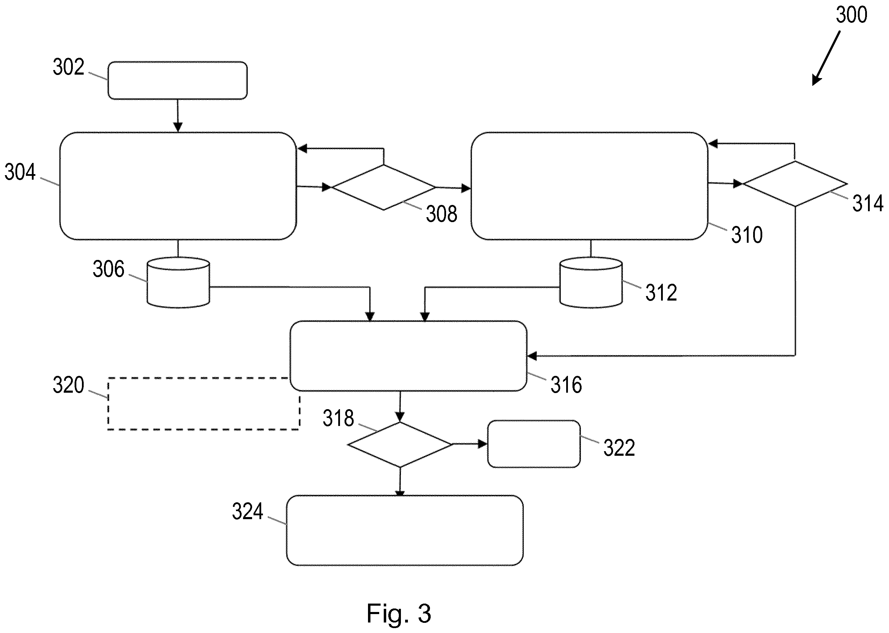

Fig. 3 illustrates a method 300 for detecting paralysis, weakness and/or numbness in a part of a body of a subject according to an embodiment. More specifically, Fig. 3 illustrates a method 300 of operating the system 100 described herein for detecting paralysis, weakness and/or numbness in a part of a body of a subject. The method 300 illustrated in Fig. 3 is a computer-implemented method. As described earlier, the system 100 comprises a processor 102. The method 300 illustrated in Fig. 3 can generally be performed by or under the control of the processor 102 of the system 100.

With reference to Fig. 3, at block 302 of Fig. 3, the subject begins using a personal care device 104 comprising a sensor 106. For example, the subject begins a routine (or session) with the personal care device 104 comprising the sensor 106. At block 304 of Fig. 3, the sensor 106 of the personal care device 104 obtains (or collects) data as the personal care device 104 is used by the subject on the first part of the body of the subject. At block 306 of Fig. 3, the data obtained by the sensor 106 of the personal care device 104 used by the subject on the first part of the body of the subject is stored in one or more memories 108 of the system 100. In the embodiment illustrated in Fig. 3, the first part of the body of the subject may comprise one side of the face, one side of the tongue, or one side of the mouth of the subject.

At block 308 of Fig. 3, the sensor 106 of the personal care device 104 used by the subject on the first part of the body of the subject detects whether the personal care device 104 switches from being used by the subject on the first part of the body (e.g. the right side of the face) of the subject to being used by the subject on the second part of the body (e.g. the left side of the face) of the subject. In the embodiment illustrated in Fig. 3, the first part of the body of the subject and the second part of the body of the subject are corresponding parts of the body of the subject on opposite sides of the body. For example, where the first part of the body of the subject may comprise one side of the face, one side of the tongue, or one side of the mouth of the subject, the second part of the body of the subject may comprise the other side of the face, the other side of the tongue, or the other side of the mouth of the subject respectively.

The sensor 106 of the personal care device 104 continues to obtain the data until the sensor 106 of the personal care device 104 detects that the personal care device 104 switches from being used by the subject on the first part of the body (e.g. the right side of the face) of the subject to being used by the subject on the second part of the body (e.g. the left side of the face) of the subject. Thus, if the sensor 106 of the personal care device 104 does not detect that the personal care device 104 switches from being used by the subject on the first part of the body (e.g. the right side of the face) of the subject to being used by the subject on the second part of the body (e.g. the left side of the face) of the subject at block 308 of Fig. 3, the sensor 106 of the personal care device 104 continues to obtain the data at block 304. The data obtained by the sensor 106 at block 304 can also continue to be sorted in one or more memories 108 of the system 100 at block 306 of Fig. 3.

On the other hand, when the sensor 106 of the personal care device 104 detects that the personal care device 104 switches from being used by the subject on the first part of the body (e.g. the right side of the face) of the subject to being used by the subject on the second part of the body (e.g. the left side of the face) of the subject, the sensor 106 of the personal care device 104 obtains (or collects) the reference data at block 310. At block 312 of Fig. 3, the reference data obtained by the sensor 106 of the personal care device 104 used by the subject on the second part of the body of the subject is stored in one or more memories 108 of the system 100.

At block 314 of Fig. 3, it may be determined whether the subject has finished using the personal care device 104 comprising a sensor 106. For example, it may be determined whether the subject has finished a routine (or session) with the personal care device 104 comprising the sensor 106. If the subject has not finished using the personal care device 104 comprising a sensor 106, the sensor 106 of the personal care device 104 continues to obtain (or collect) the reference data. The reference data obtained by the sensor 106 at block 310 can continue to be sorted in one or more memories 108 of the system 100 at block 312 of Fig. 3. On the other hand, when it is determined that the subject has finished using the personal care device 104 comprising a sensor 106, the method 300 moves to block 316.

At block 316, the processor 102 of the system acquires the data obtained by a sensor 106 of a personal care device 104 used by a subject on a first part of a body of a subject and compares the acquired data to the reference data obtained by the sensor 106 of the personal care device 104 used by the subject on a second part of the body of the subject in the manner described earlier. At block 318 of Fig. 3, it is determined whether the acquired data comprises (or it is determined whether to classify the data as comprising) an abnormality in the manner described earlier. That is, blocks 202 and 204 of Fig. 2 are performed in the manner described earlier. At block 320 of Fig. 3, in addition to comparing the acquired data to the reference data, any differences found between the acquired data and the reference data may be compared with differences found between acquired data and reference data obtained during a previous routine (or session) in which the subject used the personal care device 104 comprising the sensor 106. In this way, it is possible to reduce false alarms.

Thus, at block 318 of Fig. 3, it is determined whether the acquired data comprises (or it is determined whether to classify the data as comprising) an abnormality. If it is determined that the acquired data comprises (or it is determined to classify the data as comprising) no abnormality, then no action is taken at block 322 of Fig. 3. On the other hand, when the acquired data is determined to comprise (or is classified as comprising) an abnormality, then paralysis, weakness and/or numbness in the first part of the body of the subject or a third part of the body of the subject is detected at block 324. That is, block 206 of Fig. 2 is performed in the manner described earlier. In some embodiments, at block 324 of Fig. 3, this detection of paralysis, weakness and/or numbness may trigger the processor 102 of the system 100 to provide an output, e.g. to control at least one user interface 110 to render an output, such as any of those described earlier.

Therefore, the system 100 and methods 200, 300 described herein can be used to detect paralysis, weakness and/or numbness in a part of a body of a subject in an improved manner. The detection of paralysis, weakness and/or numbness can be valuable in healthcare as paralysis, weakness and/or numbness can be a warning sign of a potentially serious medical condition, such as a stroke, a transient ischemic attack (TIA), neurofibromatosis, brain damage, a brain or neck tumor, facial nerve damage, or other medical conditions. While the symptoms of paralysis, weakness and/or numbness often go unnoticed or are ignored by a subject, the system 100 and methods 200, 300 described herein can detect them subtly through the frequent (e.g. daily) use of a personal care device 104 by the subject.

There is also provided a computer program product comprising a computer readable medium. The computer readable medium has computer readable code embodied therein. The computer readable code is configured such that, on execution by a suitable computer or processor, the computer or processor is caused to perform the method described herein. The computer readable medium may be, for example, any entity or device capable of carrying the computer program product. For example, the computer readable medium may include a data storage, such as a ROM (such as a CD-ROM or a semiconductor ROM) or a magnetic recording medium (such as a hard disk). Furthermore, the computer readable medium may be a transmissible carrier, such as an electric or optical signal, which may be conveyed via electric or optical cable or by radio or other means. When the computer program product is embodied in such a signal, the computer readable medium may be constituted by such a cable or other device or means. Alternatively, the computer readable medium may be an integrated circuit in which the computer program product is embedded, the integrated circuit being adapted to perform, or used in the performance of, the method described herein.

There is thus provided herein an improved system 100, method 200, 300 and computer program product for detecting paralysis, weakness and/or numbness in a part of the body of a subject, which addresses the limitations associated with the existing techniques.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A system (100) for detecting paralysis, weakness and/or numbness in a part of a body of a subject, the system (100) comprising a processor (102) configured to:
acquire data obtained by a sensor (106) of a personal care device (104) used by a subject on a first part of a body of a subject, wherein the acquired data is indicative of a manner in which the subject used the personal care device (104) on the first part of the body of the subject and/or a physiological property of the first part of the body of the subject;
compare the acquired data to reference data obtained by the sensor (106) of the personal care device (104) used by the subject on a second part of the body of the subject to determine whether the acquired data comprises an abnormality, wherein the reference data is indicative of a manner in which the subject used the personal care device (104) on the second part of the body of the subject and/or a physiological property of the second part of the body of the subject; and
detect paralysis, weakness and/or numbness in the first part of the body of the subject or a third part of the body of the subject when the acquired data is determined to comprise an abnormality.

2. The system (100) as claimed in claim 1, wherein:
the first part of the body of the subject and the second part of the body of the subject are the same part of the body of the subject.

3. The system (100) as claimed in claim 1, wherein:
the first part of the body of the subject and the second part of the body of the subject are corresponding parts of the body of the subject on opposite sides of the body.

4. The system (100) as claimed in claim 3, wherein the processor (102) is configured to:
detect partial paralysis, weakness and/or numbness in the first part of the body of the subject when the acquired data is determined to comprise an abnormality.

5. The system (100) as claimed in any of the preceding claims, wherein:
the third part of the body of the subject is a different part of the body of the subject to the first and second parts of the body of the subject.

6. The system (100) as claimed in any of the preceding claims, wherein the reference data comprises previous and/or subsequent data obtained by the sensor (106) of the personal care device (104) used by the subject on the second part of the body of the subject.

7. The system (100) as claimed in any of the preceding claims, wherein the processor (102) is configured to:
determine that the acquired data comprises an abnormality if the acquired data differs from the reference data by more than a predefined amount.

8. The system (100) as claimed in any of the preceding claims, wherein the processor (102) is configured to:
if paralysis, weakness and/or numbness is detected in the first part of the body of the subject, output any one or more of: an indication of the detected paralysis, weakness and/or numbness, an indication that the subject is at an increased risk of a medical condition, and a request for information from the subject.

9. The system (100) as claimed in any of the preceding claims, the system (100) comprising:
the personal care device (104) comprising the sensor (106),
wherein the sensor (106) is configured to:
obtain the data while the subject uses the personal care device (104) on the first part of the body of the subject; and
obtain the reference data while the subject uses the personal care device (104) on the second part of the body of the subject.

10. The system (100) as claimed in claim 9, wherein the personal care device (104) comprises the processor (102).

11. The system (100) as claimed in any of the preceding claims, wherein the manner in which the subject uses the personal care device (104) comprises any one or more of:
the manner in which the subject holds the personal care device (104);
the direction in which the subject uses the personal care device (104);
the manner in which the subject moves the personal care device (104);
the speed at which the subject moves the personal care device (104);
the pressure or force with which the subject uses the personal care device (104); and
the orientation at which the subject uses the personal care device (104).

12. The system (100) as claimed in any of the preceding claims, wherein the physiological property comprises any one or more of:
a muscle tension;
a muscle elasticity;
a skin morphology; and
a skin and/or tongue orientation.

13. A system (100) as claimed in any of the preceding claims, wherein the paralysis, weakness and/or numbness comprises any one or more of:
a facial paralysis, weakness and/or numbness;
a tongue paralysis, weakness and/or numbness; and
a hand paralysis, weakness and/or numbness.

14. A method (200, 300) for detecting paralysis, weakness and/or numbness in a part of a body of a subject, the method (200, 300) comprising:
acquiring (202, 304) data obtained by a sensor of a personal care device used by a subject on a first part of a body of a subject, wherein the acquired data is indicative of a manner in which the subject used the personal care device on the first part of the body of the subject and/or a physiological property of the first part of the body of the subject;
comparing (204, 316) the acquired data to reference data obtained by the sensor of the personal care device used by the subject on a second part of the body of the subject to determine whether the acquired data comprises an abnormality, wherein the reference data is indicative of a manner in which the subject used the personal care device on a second part of the body of the subject and/or a physiological property of the second part of the body of the subject; and
detecting (206, 324) paralysis, weakness and/or numbness in the first part of the body of the subject or a third part of the body of the subject when the acquired data is determined to comprise an abnormality.

15. A computer program product comprising a computer readable medium, the computer readable medium having computer readable code embodied therein, the computer readable code being configured such that, on execution by a suitable computer or processor, the computer or processor is caused to perform the method as claimed in claim 14.
